# EUROPEAN PATENT APPLICATION

(11) **EP 1 352 657 A1**
(43) Date of publication of application: **15.10.2003**
(21) Application number: 01998362.6
(22) Date of filing: 28.11.2001
(51) Int. Cl.: A61K 38/40, A61K 38/21, A61P 43/00, A61P 31/20, A61P 1/18, A61P 35/00

(54) **INTERFERON THERAPEUTIC EFFECT-POTENTIATING AGENTS**

(30) Priority: 29.11.2000 JP 2000362813
(71) Applicant: Morinaga Milk Industry Co., Ltd., Minato-ku, Tokyo 108-8384 (JP)
(72) Inventor: TANAKA, Katsuaki c/o Yokohama City Univ.Med.Ctr., Minami-ku Yokohama-shi Kanagawa 228-0004 (JP); HAYASAWA, Hirotoshi c/o Morinaga Milk Ind.Co.,Ltd., Zama-shi Kanagawa 228-0004 (JP); TERAGUCHI, Susumu c/o Morinaga Milk Ind. Co.,Ltd., Zama-shi Kanagawa 228-0004 (JP); YAMAUCHI, Koji c/o Morinaga Milk Ind. Co., Ltd., Zama-shi Kanagawa 228-0004 (JP)
(74) Representative: Poulin, Gérard
(86) International application number: JP0110383
(87) International publication number: WO02043752

(57) **Abstract**

The present invention relates to an interferon therapeutic effect enhancer that contains lactoferrin as its active ingredient; the use of lactoferrin for the production of an interferon therapeutic effect enhancer; a pharmaceutical composition having interferon and lactoferrin as its active ingredients; and, a disease treatment method that includes administration of said pharmaceutical composition, said interferon therapeutic effect enhancer and said pharmaceutical composition being able to improve the therapeutic effects of interferon against various diseases without the occurrence of adverse side effects.

## Description

### BACKGROUND OF THE INVENTION

### Field of the Invention

The present invention relates to an interferon therapeutic effect enhancer that contains lactoferrin as its active ingredient. More particularly, the present invention relates to an agent that enhances the effectiveness of interferon treatment against various diseases which has for its active ingredient metal unsaturated lactoferrin, metal saturated lactoferrin or apolactoferrin (and these may be collectively described as lactoferrins hereinafter). Moreover, the present invention relates to the use of said lactoferrin, a pharmaceutical composition having interferon and lactoferrin as its active ingredients, and a disease treatment method using said pharmaceutical composition.

### Description of the Related Art

Interferon is widely used in the treatment of diseases such as chronic hepatitis type B, chronic hepatitis type C, kidney cancer and multiple myeloma. However, its efficacy is not always high, and in cases exhibiting high virus counts in particular, interferon resistance has been observed, resulting in the problem of being unable to obtain adequate therapeutic effects. For example, the very effective rate of interferon α2b against chronic hepatitis type C is only 29.8%, while that against multiple myeloma is only 20.8% (Japan Pharmaceutical Information Center, ed., Year 2000 Edition, "Medicines and Drugs in Japan", page 275, Yakugyo Jiho Co., Ltd., 1999).

In consideration of the above circumstances, the object of the present invention is to provide an inexpensive enhancer of the therapeutic effects of interferon against various diseases without causing adverse side effects, the use of said enhancer as an active ingredient, a pharmaceutical composition having for its active ingredients said enhancer and interferon, and a treatment method that uses said pharmaceutical composition.

As a result of conducting extensive research on drugs that enhance the efficacy of interferon, the inventors of the present invention focused their attention on lactoferrin.

Lactoferrin is a harmless and naturally-occurring iron-binding protein (capable of binding two iron ions per molecule) that is contained in tears, saliva, peripheral blood, mother's milk and so forth. Bovine lactoferrin has a molecular weight of 86,000, while human lactoferrin has a molecular weight of 88,000 (Kazutomo Imabori and Tamio Yamakawa, ed., "Dictionary of Biochemistry", 2nd edition, page 1390, Tokyo Kagaku Dojin Co., Ltd., 1990).

Lactoferrin has been reported to exhibit antimicrobial action against harmful microorganisms such as Escherichia coli, Candida species and Chlostridium species (Journal of Pediatrics, Vol. 94, page 1, 1979), be effective in fixating useful bacteria such as *Lactobacillus bifidus* and lactic acid bacillus in human and animal intestine (Japanese Patent No. 2532911), be a *Lactobacillus bifidus* growth factor (Japanese Unexamined Patent Application, First Publication No. Hei 2-225419), exhibit antiviral action against hepatitis C virus in vitro (Noriyuki Katoh, ed., "Hepatitis C Virus" , IPC Co., Ltd., page 101, 2000), and have clinical therapeutic effects for chronic hepatitis type C patients following oral administration of lactoferrin (Bio Industry, Vol. 17, page 45, 2000).

However, these therapeutic effects are limited to cases having low virus counts, and effects are not observed in cases having high virus counts that are resistant to interferon (Japanese Journal of Cancer Research, Vol. 90, page 367, 1999).

Moreover, lactoferrin has also been disclosed as a lactoprotein having various actions such as immunoactivating action (Japanese Unexamined Patent Application, First Publication No. Hei 7-179355), cell growth action (Japanese Unexamined Patent Application, First Publication No.Hei 6-48955), antitumor action (Cancer Research, Vol. 54, page 2310, 1994), action as an antirheumatic agent applied to disease therapeutic agents (Japanese Unexamined Patent Application, First Publication No. Hei 5-186368), and action as a liver function ameliorant against drug-induced chronic liver disease (WO 00/06192).

However, lactoferrin is not known to have action that enhances the efficacy of interferon in the treatment of various diseases exemplified by chronic viral hepatitis, and there are no reports of such in the literature.

### SUMMARY OF THE INVENTION

The inventors of the present invention found that administration of lactoferrin remarkably enhances the therapeutic effects of interferon against various diseases as compared with not administering lactoferrin, thereby leading to completion of the present invention. In particular, as was previously stated, the unexpected finding was obtained in which concomitant use of interferon and lactoferrin was observed to enhance therapeutic effects against viral diseases associated with low therapeutic effects or diseases that are resistant to interferon that exhibit a high virus count when interferon or lactoferrin is used alone.

A first aspect of the present invention for solving the above problems is an interferon therapeutic effect enhancer that contains lactoferrin as its active ingredient.

In addition, a second aspect of the present invention for solving the above problems is an interferon therapeutic effect enhancer for treatment of chronic viral hepatitis that contains lactoferrin as its active ingredient.

In addition, a third aspect of the present invention for solving the above problems is the use of lactoferrin for production of an interferon therapeutic effect enhancer.

In addition, a fourth aspect of the present invention for solving the above problems is the use of lactoferrin for producing an interferon therapeutic effect enhancer for treatment of chronic viral hepatitis.

In addition, a fifth aspect of the present invention for solving the above problems is a pharmaceutical composition having interferon and lactoferrin as its active ingredients. A preferable mode of said pharmaceutical composition is that it be for chronic hepatitis type B, chronic hepatitis type C, kidney cancer and multiple myeloma.

In addition, a sixth aspect of the present invention for solving the above problems is a disease treatment method that includes administration of said pharmaceutical composition.

### DETAILED DESCRIPTION OF THE PREFERRED EMBODIMENTS

The following provides a detailed explanation of the present invention.

The lactoferrins used as the active ingredient of the present invention may be commercially available products or lactoferrins isolated by ordinary methods such as ion exchange chromatography from the colostrum, transitional milk, mature milk, or their processed products in the form of skim milk or whey and so forth of mammals (including humans, cows, water buffaloes, horses, goats and sheep). Moreover, they may also be apolactoferrins, in which iron has been removed from lactoferrin by ordinary methods, metal unsaturated lactoferrins or metal saturated lactoferrins in which a portion or all of a metal such as iron, copper, zinc or manganese is chelated to apolactoferrin.

In addition, although naturally-occurring human lactoferrin cannot be produced in large volume, human lactoferrin produced from recombinant fungi or recombinant dairy cows (transgenic cows) obtained by recombinant DNA technology can also be used in the present invention.

The interferon therapeutic effect enhancer of the present invention is administered by blending lactoferrin and other components and preparing in various modes in accordance with known methods. Specific examples of preparations include tablets (including sugar-coated tablets, coated tablets and buccal tablets), powders, capsules (including soft capsules), granules (including coated granules), pills, troches, liquids as well as their pharmaceutically acceptable sustained-release preparations, oral preparations, enteral preparations and injections.

The above preparations are prepared in the form of pharmaceutical compositions together with pharmacologically acceptable carriers, excipients, disintegration agents, lubricants, colorants and so forth in compliance with known pharmaceutical production methods.

Examples of carriers and excipients used in these preparations include lactose, glucose, sucrose, mannitol, potato starch, corn starch, calcium carbonate, calcium phosphate, calcium sulfate, crystalline cellulose, licorice powder and gentiana powder. In addition, examples of binders include starch, gelatin, syrup, polyvinyl alcohol, polyvinyl ether, polyvinyl pyrrolidone, hydroxypropyl cellulose, ethyl cellulose, methyl cellulose and carboxymethyl cellulose.

In addition, examples of disintegration agents include starch, agar, powdered gelatin, sodium carboxymethyl cellulose, calcium carboxymethyl cellulose, crystalline cellulose, calcium carbonate, sodium bicarbonate and sodium alginate.

Moreover, examples of lubricants include magnesium stearate, hydrogenated vegetable oils and macrogol, while examples of colorants include Red Dye No. 2, Yellow Dye No. 2 and Blue Dye No. 1 that are allowed to be added to pharmaceuticals.

Tablets and granules may also be coated with sucrose, hydroxypropyl cellulose, shellac, gelatin, sorbitol, glycerin, ethyl cellulose, hydroxypropyl cellulose, hydroxypropylmethyl cellulose, polyvinyl pyrrolidone, cellulose phthalate acetate, hydroxypropylmethyl cellulose phthalate, methyl methacrylate, methacrylate polymers and so forth.

The interferon therapeutic effect enhancer of the present invention preferably contains at least 1 mg of lactoferrin as its active ingredient per 1 g of pharmaceutical composition, and although varying according to age, symptoms and so forth, the dosage is preferably 20 mg to 15 g, and particularly preferably 100 mg to 10 g, per day administered at the rate of at least 1 mg per 1 kg of human body weight.

Moreover, there are no particular restrictions on the form of the interferon therapeutic effect enhancer of the present invention, and products in the form of foods and drinks represented by special health foods are included.

In addition, the time at which the interferon therapeutic effect enhancer of the present invention is administered may be prior to, simultaneous to or after the start of interferon therapy.

Interferon (IFN) for which efficacy is enhanced by the interferon therapeutic effect enhancer of the present invention may be any interferon among naturally-occurring interferon α, interferon β or genetic recombinant interferon α2a or interferon α2b, or their preparations.

Although the target diseases for administration of the interferon therapeutic effect enhancer of the present invention may be chronic hepatitis type B, chronic hepatitis type C, kidney cancer, multiple myeloma and so forth for which interferon is recognized to be therapeutically effective, in particular consideration of the high prevalence of chronic viral hepatitis among all diseases in modern society, using the interferon therapeutic effect enhancer of the present invention for the treatment of chronic viral hepatitis clearly described as a second aspect of the present invention is the most effective.

In addition, since the interferon therapeutic effect enhancer of the present invention is extremely effective against interferon-resistant viral diseases, it is preferable to select interferon-resistant chronic hepatitis type C (and more specifically, chronic hepatitis type C in which the type of infecting virus is HCV (hepatitis C virus) of genotype 1b, or chronic hepatitis type C in which HCV-RNA level indicates a high virus count of at least 100 KIU/ml) for the target disease for administration.

In addition, pharmaceutical compositions of the present invention may contain both interferon and lactoferrin as their active ingredients. Said pharmaceutical compositions are preferably used for chronic hepatitis type B, chronic hepatitis type C, kidney cancer and multiple myeloma. Since the pharmaceutical compositions of the present invention are extremely effective against interferon-resistant viral diseases, it is preferable to select interferon-resistant chronic hepatitis type C (and more specifically, chronic hepatitis type C in which the type of infecting virus is HCV (hepatitis C virus) of genotype 1b, or chronic hepatitis type C in which HCV-RNA level indicates a high virus count of at least 100 KIU/ml) for the target disease for administration.

The pharmaceutical compositions of the present invention preferably contain at least 1 mg of active ingredient in the form of lactoferrin per 1 g of pharmaceutical composition, and although varying according to age, symptoms and so forth, the dosage is preferably 20 mg to 15 g, and particularly preferably 100 mg to 10 g, per day administered at the rate of at least 1 mg per 1 kg of human body weight.

The pharmaceutical compositions of the present invention can be blended with interferon, lactoferrin and other ingredients, and can be prepared in various forms using methods similar to the interferon therapeutic effect enhancer.

Next, the following provides a detailed explanation of the present invention by indicating test examples.

### Test Example 1

This test was conducted in order to investigate the enhancing action of lactoferrin on the therapeutic effect of interferon.

### (1) Sample Preparation

Lactoferrin (Morinaga Milk Industry Co., Ltd.) was used for the test sample.

### (2) Subjects

The subjects consisted of a total of 12 patients with chronic hepatitis type C in which the type of infecting virus was HCV (hepatitis C virus) of genotype 1b and chronic hepatitis type C in which the HCV-RNA level exhibited a virus count of at least 100 KIU/ml who were randomly divided into two groups consisting of an interferon only treatment group comprised of 6 patients and an interferon and lactoferrin concomitant treatment group comprised of 6 patients.

### (3) Test Method

Both groups were administered 10,000,000 units per day of α-interferon for 2 weeks, and then administered the same at the rate of 3 times per week for a total of 24 weeks.

In addition, lactoferrin was orally administered daily at 1.8 g per day for 6 months during and after administration of interferon to subjects of the concomitant treatment group only.

Blood samples were collected over time after the start of administration, and serum glutamic-pyruvic transaminase (abbreviated as GPT) levels as well as blood virus levels were measured.

A comparative study was conducted on the therapeutic effects in the above two groups based on these results. Furthermore, subjects in which the virus had been eradicated and liver function (GPT values) had been normalized at 6 months after completion of interferon therapy were judged as exhibiting very effective therapeutic effects, and subjects in which, although virus was not detected during interferon therapy, the virus was redetected at 6 months after completion of interferon therapy and GPT values were normalized were judged as exhibiting effective therapeutic effects. Other subjects were judged as exhibiting ineffective therapeutic effects.

### (4) Test Results

Enhancement of interferon therapeutic effects by lactoferrin was as described below.

The efficacy rates of therapeutic effects for each treatment group were such that, in contrast to all six subjects of the interferon only treatment group being judged as ineffective for an efficacy rate of 0%, 4 of 6 subjects of the concomitant treatment group were judged as effective or better (of which 2 were effective and 2 were very effective) for an efficacy rate of 67%.

Namely, lactoferrin was observed to demonstrate action that enhances the therapeutic effects of interferon.

In addition, the results of this test also indicated that concomitant use of lactoferrin and interferon is extremely effective in the treatment of cases that are resistant to interferon.

Furthermore, when a concomitant treatment test was conducted in the same manner as above on 2 patients with chronic hepatitis type C in which the type of infecting virus was HCV (hepatitis C virus) of genotype 2a, and the HCV-RNA count exhibited a value of less than 100 KIU/ml, the results were observed to be very effective for both patients.

Next, although the following provides a more detailed explanation of the present invention by indicating its examples, the present invention is not limited to the following examples.

### EXAMPLES

### Example 1 - Preparation of Tablets Blended with Bovine Lactoferrin

Tablets of an interferon therapeutic effect enhancer for the treatment of chronic viral hepatitis having the following composition were produced according to the method described below.

| | |
|---|---|
| Bovine lactoferrin (Milei) | 20.0 (%) |
| Powdered *Bifidobacterium longum* (Morinaga Milk) | 15.0 |
| Lactulose (Morinaga Milk) | 20.0 |
| Reduced maltose (Towa Kasei Kogyo) | 27.6 |
| Sweetener (Nikken Chemicals) | 15.2 |
| Lubricant (Riken Vitamin) | 1.8 |
| Fragrance (Hasegawa Koryo) | 0.4 |

A mixture of bovine lactoferrin, powdered *Bifidobacterium longum,* lactulose, reduced maltose, sweetener, lubricant and fragrance were molded into tablets in accordance with ordinary methods to obtain tablets.

### Example 2 - Preparation of Tablets Blended with Bovine Lactoferrin

Tablets of an interferon therapeutic effect enhancer having the following composition were produced according to the method described below.

| | |
|---|---|
| Bovine lactoferrin (Milei) | 40.0 (%) |
| Lactose (Morinaga Milk) | 18.5 |
| Corn starch (Nissei Seifun) | 30.7 |
| Magnesium stearate (Taihei Kagaku Sangyo) | 1.4 |
| Calcium carboxymethyl cellulose (Gotoku Yakuhin) | 9.4 |

Sterile purified water was uniformly mixed while suitably adding to a mixture of bovine lactoferrin, lactose, corn starch and calcium carboxymethyl cellulose followed by drying for 3 hours at 50°C and mixing in magnesium stearate to the resulting dried product followed by the formation of tablets in accordance with ordinary methods to obtain tablets.

### Example 3 - Preparation of Syrup Blended with Bovine Lactoferrin

Syrup of an interferon therapeutic effect enhancer having the following composition was produced according to the method described below.

| | |
|---|---|
| Bovine lactoferrin (Milei) | 8.0 (%) |
| Fructose-glucose sugar solution (Sansho Kogyo) | 12.4 |
| Citric acid (Ueno Seiyaku) | 0.2 |
| Sodium citrate (Maruzen Seiyaku) | 0.2 |
| Calcium carboxymethyl cellulose (Gotoku Yakuhin) | 0.2 |
| Purified water (Otsuka Seiyaku) | 79.0 |

### Example 4 - Preparation of Bovine Lactoferrin Capsules

Capsules of interferon therapeutic effect enhancer were produced in the manner described below.

600 g of lactose (Wako Pure Chemical Industries), 400 g of corn starch (Nissei Seifun), 400 g of crystalline cellulose (Wako Pure Chemical Industries) and 600 g of bovine lactoferrin (Milei) were sized with a 50 mesh sieve (Yamato Kagaku), transferred to a 0.5 mm thick polyethylene bag and mixed by inverting followed by filling the above powder into capsules (Japan Elanco; No. 1 gelatin capsules, Op. Yellow No. 6 body, empty weight: 75 mg) to a content weight of 275 mg using a fully automated capsule filling machine (Cesere Pedini, press type) to obtain 7,000 capsules containing 82 mg of lactoferrin.

### Example 5 - Preparation of Human Lactoferrin Powder

A powder of an interferon therapeutic effect enhancer was produced in the manner described below.

100 g of human lactoferrin powder (Milei) pre-sized with a No. 6 sieve (luchi Seieido) and 500 g of lactose (Morinaga Milk) were mixed in a mortar followed by mixing in 400 g of lactose (Morinaga Milk) pre-sized with a No. 5 sieve (Iuchi Seieido) followed by again sizing the entire amount with a No. 5 sieve and packaging into individual packs containing 5 g per pack with a packaging machine (Tokyo Shokai; OMP-90A) to obtain 200 packs of 10% human lactoferrin powder.

As has been described in detail above, the present invention demonstrates the following effects, and can be used in the fields of pharmaceuticals, health foods and so forth.
(1) Since the present invention contains protein originating in milk and other foods as its main ingredient, it is economical and causes hardly any adverse side effects even when continuously ingested for a long period of time.
(2) The present invention has the action of enhancing the efficacy of interferon in the treatment of various diseases exemplified by chronic viral hepatitis.
(3) The present invention is very effective in the treatment of patients with chronic hepatitis type C who are resistant to interferon.

## Claims

1. An interferon therapeutic effect enhancer comprising lactoferrin as its active ingredient.

2. An interferon therapeutic effect enhancer for the treatment of chronic viral hepatitis, comprising lactoferrin as its active ingredient.

3. The use of lactoferrin for the production of an interferon therapeutic effect enhancer.

4. The use of lactoferrin for the production of an interferon therapeutic effect enhancer for the treatment of chronic viral hepatitis.

5. A pharmaceutical composition comprising interferon and lactoferrin as its active ingredients.

6. The pharmaceutical composition according to claim 5 that is for chronic hepatitis type B.

7. The pharmaceutical composition according to claim 5 that is for chronic hepatitis type C.

8. The pharmaceutical composition according to claim 5 that is for kidney cancer.

9. The pharmaceutical composition according to claim 5 that is for multiple myeloma.

10. A method of treating a disease comprising administration of the pharmaceutical composition according to claim 5.
